(19) Europäisches Patentamt
European Patent Office
Office européen des brevets

(11) Veröffentlichungsnummer: **0 227 054 B1**

(12) **EUROPÄISCHE PATENTSCHRIFT**

(45) Veröffentlichungstag der Patentschrift: **18.09.91**

(51) Int. Cl.[5]: **C08F 257/02**, C08F 291/00, C07K 17/08, G01N 33/546

(21) Anmeldenummer: **86117694.9**

(22) Anmeldetag: **18.12.86**

# (54) Dispersionspolymere, Verfahren zu ihrer Herstellung und ihre Verwendung.

(30) Priorität: **21.12.85 DE 3545595**

(43) Veröffentlichungstag der Anmeldung:
**01.07.87 Patentblatt 87/27**

(45) Bekanntmachung des Hinweises auf die Patenterteilung:
**18.09.91 Patentblatt 91/38**

(84) Benannte Vertragsstaaten:
**AT BE CH DE ES FR GB IT LI LU NL SE**

(56) Entgegenhaltungen:
**EP-A- 0 080 614**
**EP-A- 0 106 769**

(73) Patentinhaber: **BEHRINGWERKE Aktiengesellschaft**
**Postfach 1140**
**W-3550 Marburg 1(DE)**

(72) Erfinder: **Kapmeyer, Wolfgang, Dr.**
**Reinhardswaldstr. 5**
**W-3550 Marburg 7(DE)**
Erfinder: **Rinno, Helmut, Dr.**
**Goethestr. 38**
**W-6238 Hofheim am Taunus 7(DE)**

(74) Vertreter: **Becker, Heinrich Karl Engelbert, Dr. et al**
**HOECHST AKTIENGESELLSCHAFT Central Patent Department P.O. Box 80 03 20**
**W-6230 Frankfurt am Main 80(DE)**

**EP 0 227 054 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Dispersionspolymere, Verfahren zu ihrer Herstellung und ihre Verwendung, wobei die Dispersionspolymere aus Latexpartikeln bestehen, deren äußere Schicht ein Copolymerisat aus Vinylmonomeren bildet, von denen eines eine hydroxylgruppentragende N-Alkyl-Acrylamid-Verbindung beziehungsweise ein entsprechender Methacrylsäureester ist. Man gewinnt daraus biologisch aktive Dispersionspolymere, indem man biologisch aktive Substanzen, die über freie Aminogruppen verfügen, an reaktive, von der Aldehydfunktion abgeleitete Gruppen auf der Oberfläche der erfindungsgemäßen Dispersionspolymerteilchen bindet. Diese biologisch aktiven Dispersionspolymere sind geeignet für serologische oder immunologische Bestimmungsverfahren.

Es ist bekannt, die Empfindlichkeit serologischer oder immunologischer Bestimmungsverfahren durch die Verwendung von Indikator- oder Trägerteilchen zu erhöhen, die mit dem entsprechenden immunologischen Reagenz beladen sind. Als Trägermaterial können zum Beispiel rote Blutkörperchen oder Zellen einer Zellkultur verwendet werden. Auch Latexpartikel mit einem Durchmesser von 0,02 bis 5 $\mu$m werden hierfür eingesetzt.

Es ist ferner bekannt, Polyhydroxy-Verbindungen, wie Zucker oder Dextrane, zum Nachbeschichten eines fertig auspolymerisierten Latex zu verwenden (EP 0001223, Hoffmann La Roche). Derartige Latices sind nicht stabil gegenüber detergentienhaltigen Puffern, da die adsorptiv gebundenen Polyhydroxy-Verbindungen durch derartige Puffer vom Latex abgelöst werden können.

Als Stand der Technik sind aus der Europäischen Patentanmeldung EP-A 0 080 614 Latexpartikel bekannt, die über Säureamidgruppen gebundene Acetal-Funktionen enthalten. Im folgenden wird diese Anmeldung als Stand der Technik bezeichnet. In einem wässrigen Medium vorgefertigte Latexkerne werden mit Vinylmonomeren, die über Säureamidgruppen gebundene Acetal-Funktionen enthalten, angequollen und diese Vinylmonomere, die ausreichend wasserunlöslich sein müssen, werden dann zusammen mit weiteren Monomeren, die hydrophiler oder ionischer Natur sein können, copolymerisiert. Derartige Reagenzien lassen sich zur nephelometrischen Bestimmung von C-reaktivem Protein einsetzen. Dazu werden Serumproben hoch mit Puffer verdünnt, normalerweise 1:1000, wodurch störende Serumproteine, die anderenfalls zu falschpositiven oder falschnegativen Ergebnissen führen würden, vernachlässigbar werden. Diese Arbeitsweise ist möglich, weil für diagnostische Zwecke im allgemeinen Konzentrationen an C-reaktivem Protein von mehr als 5 mg/l vorliegen müssen. Will man aber die Konzentration von Spurenproteinen im Bereich von 1 $\mu$g/l bis 50 $\mu$g/l messen, dann dürfen die Proben nicht entsprechend hoch mit Puffer verdünnt werden, weil sonst die Konzentration des nachzuweisenden Proteins so gering wird, daß die Nachweisempfindlichkeit nicht ausreicht.

Eine Steigerung der Nachweisempfindlichkeit bei Latexpräparationen nach dem Stand der Technik ist jedoch nicht ohne weiteres möglich und liefert zum Beispiel für die Bestimmung von IgE keine zufriedenstellend funktionierenden Tests. Der Versuch, die Empfindlichkeit zu steigern, führt dazu, daß die Signale für die Messung einer Referenzkurve schon nach relativ kurzer Zeit unspezifisch derart ansteigen, daß eine Auswertung nicht mehr möglich ist. Es gibt keine abgestufte Referenzkurve mehr. Die Ursache liegt darin, daß die einzelnen Partikel eines derartigen, instabilen Reagenzes sich ohne die Anwesenheit von Antigen agglutinierend zusammenballen und so zu einer Erhöhung des Streulichtsignals oder der Extinktion führen.

Es wurde nun überraschend gefunden, daß die geschilderten Nachteile des Standes der Technik überwunden werden können, wenn man Trägerpartikel verwendet, die dadurch hergestellt sind, daß vorgefertigte Latexkerne in einem wässrigen Medium mit Acryl- oder Methacrylmonomeren, die über Säureamidgruppen gebundene Acetal-Funktionen enthalten, zusammen mit Acryl- oder Methacrylmonomeren, die eine oder mehrere Hydroxylgruppen tragen, copolymerisiert werden.

Gegenstand der Erfindung sind somit Dispersionspolymere, die Trägerteilchen enthalten, welche aus Latex in einem wässrigen Medium bestehen, auf deren Oberfläche sich ein Copolymerisat befindet, das Monomeren mit endständigen Acetalen der Formel I

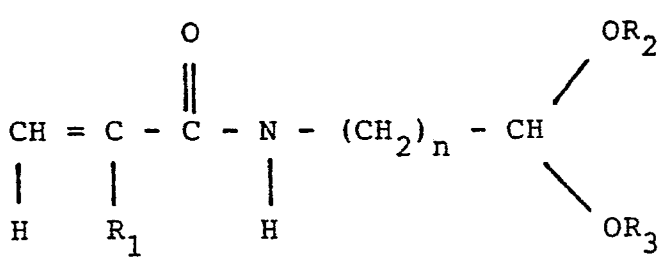

I

worin

n = 1 - 6;

$R_1$ = H, $CH_3$ und

$R_2$ und $R_3$ gleich oder unterschiedlich sind mit

$R_2$, $R_3$ = $-(CH_2)m - CH_3$, m = 0 - 7 oder

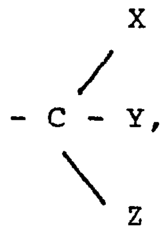

wobei X, Y, Z = $(CH_2)_pCH_3$ und p = 1 - 3 sind, mit X, Y, Z gleich oder unterschiedlich

und Monomeren der Formel II

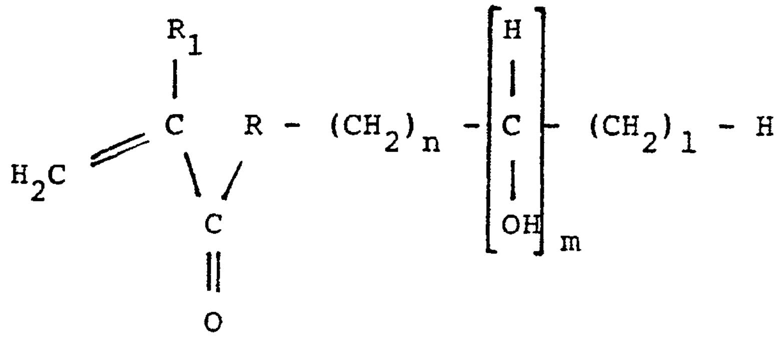

II

worin

R = 0,NH; n = 1 - 3; m = 1 - 4; 1 = 0 - 4

$R_1$ = H, $CH_3$

bedeuten, enthält.

Die erfindungsgemäßen Dispersionen (beziehungsweise Latices) können durch Copolymerisation auf üblichen, herkömmlichen Latexpartikeln als Saatdispersion hergestellt werden, die mittels bekannter Verfahren als Homo- oder Copolymerisate von Monomeren erhalten werden können.

Die Latex-Partikel, die als Saatdispersion für die erfindungsgemäßen Dispersionspolymere eingesetzt werden, sollen nicht filmbildende Polymerisate sein. Unter "nicht-filmbildend" werden Polymer-Latexteilchen verstanden, die keinen Film unter den hier in Frage kommenden Anwendungsbedingungen bilden und nicht zusammenfließen. Polymerisate aus carbocyclischen aromatischen Monovinylidenmonomeren, wie Styrol, Vinyltoluol und Vinylnaphthalin sowie Mischungen dieser Monomeren untereinander und/oder mit Methylmethacrylat und Acrylnitril sind bevorzugt. Besonders bevorzugte Saatdispersionen sind Polystyrol-Latices.

Zur Herstellung des erfindungsgemäßen Dispersionspolymeres wird grundsätzlich ein vorgefertigter Latex mit Partikeldurchmesser von 0,02 bis 2 µm, vorzugsweise 0,05 bis 0,5 µm, mit etwa 20-80 % der Menge eines Emulgators versetzt, der zur maximalen monomolekularen Bedeckung der Latexoberfläche notwendig wäre. Messungen zur Bestimmung der Emulgatormenge, die zu einer maximalen Bedeckung der Latexoberfläche führt, werden mit Hilfe eines Tensiometers durchgeführt. Sie sind zum Beispiel von I. Phrma und S.-R. Chen im Journal of Colloid and Interface Science, Vol. 74 (1979), S. 90-102 und erstmalig von S.H. Maron, M.E. Elder und I.N. Ulevitch im Journal of Colloid Interface Sciences, Vol. 9 (1954), S. 89-104, publiziert worden.

In Frage kommende Emulgatoren sind beispielsweise Polyglycolether mit langkettigen, aliphatischen Alkoholen, die vorzugsweise 10-20 Kohlenstoffatome aufweisen, oder Alkylphenol, deren Alkylrest vorzugsweise 6-12 Kohlenstoffatome enthält, oder von Dialkylphenolen oder Trialkylphenolen, deren Alkylreste vorzugsweise verzweigte Alkylreste mit jeweils 3-12 Kohlenstoffatomen darstellen. Beispiele hierfür sind Umsetzungsprodukte von Äthylenoxyd mit Laurylalkohol, Stearylalkohol, Oleylalkohol, Kokosfettalkohol,

Octylphenol, Nonylphenol, Diisopropylphenol, Triisopropylphenol, Di-t-butylphenol und Tri-t-butylphenol. Reaktionsprodukte des Ethylenoxids mit Polypropylenglykol oder Polybutylenglykol sind ebenfalls geeignet.

Von den ionischen Emulgatoren sind vor allem anionische Emulgatoren geeignet, insbesondere Alkali- oder Ammoniumsalze von Alkylsulfonaten, Arylsulfonaten oder Alkylarylsulfonaten sowie von den entsprechenden Sulfaten, Phosphaten oder Phosphonaten, die gegebenenfalls Oxyethylen-Einheiten zwischen dem jeweiligen Kohlenwasserstoffrest und der anionischen Gruppe aufweisen. Beispiele hierfür sind Natriumdodecylsulfat, Natriumlaurylsulfat, Natriumoctylphenolglykolethersulfat, Natrumdodecylbenzolsulfonat, Natriumlauryldiglykolsulfat, Ammonium-tri-t-butylphenolpentaglykolsulfat und Ammonium-tri-t-butylphenoloctaglykolsulfat. Bevorzugt eingesetzt wird Natriumdodecylsulfat.

Die Polymerisation erfolgt nach an sich bekannten Verfahren in Gegenwart eines radikalbildenden Initiators, z. B. einer Peroxid-Verbindung oder einer aliphatischen Azoverbindung. Der einzusetzende Initiator ist vorzugsweise wasserlöslich; er wird in einer Menge von 0,05 bis 10 Gew. %, vorzugsweise 0,1 bis 3 Gew. % eingesetzt (bezogen auf die Gesamtmenge der Monomeren). Bekannte radikalbildende Initiatoren sind zum Beispiel Wasserstoffperoxid, Alkali- oder Ammoniumsalze der Peroxydischwefelsäure oder der Peroxydiphosphorsäure, zum Beispiel Natriumperoxydisulfat, Kaliumperoxydisulfat und Ammoniumperoxydisulfat, ferner Alkylhydroperoxide wie t-Butylhydroperoxid, Dialkylperoxide wie Di-t-butylperoxid, Diacylperoxide wie Diacetylperoxid, Dilauroylperoxid und Dibenzoylperoxid sowie Azodiisobuttersäurenitril, Azodicarbonamid und Azogamma,gamma'-bis(4-cyanvaleriansäure). Bevorzugt eingesetzt werden die Alkali- oder Ammoniumsalze der Peroxidischwefelsäure, wie Natrium-, Kalium- und Ammoniumperoxidisulfat.

Der Initiator wird gegebenenfalls zusammen mit einem Reduktionsmittel eingesetzt, insbesondere mit einem Alkalisalz oder Erdalkalisalz einer reduzierend wirkenden schwefelhaltigen Säure; vorzugsweise eignen sich Sulfite, Bisulfite, Pyrosulfite, Dithionite, Thiosulfate und Formaldehydsulfoxylate. Gleichfalls verwendbar sind auch Glukose und Ascorbinsäure.

Zu der Saatdispersion, die Emulgator und Radikalstarter enthält, wird die Monomerenmischung aus Hydroxylgruppen enthaltendem Monomer der Formel II und Acetalgruppen enthaltendem Monomer der Formel I unter Rühren zugetropft. Die Temperatur der Dispersion liegt zwischen + 10° und + 120° C, vorzugsweise zwischen + 50° und + 90° C.

Als Hydroxylgruppen enthaltendes Monomer eignen sich die Polyhydroxyverbindungen der Formel II. Vorzugsweise werden Mono- oder Dihydroxyalkylacryl- oder methacrylverbindungen eingesetzt. Besonders bevorzugt sind N-2,3-Dihydroxypropylmethacrylamid, N-2-Hydroxypropylmethacrylamid, 2-Hydroxypropylmethacrylat sowie die entsprechenden Acrylverbindungen.

Als Acetalgruppen enthaltende Monomere werden die Verbindungen der Formel I eingesetzt, bevorzugt verwendet man Acryl- oder Methacrylamidoalkylaldehyd-di-alkylacetal mit Alkyl = $C_2$ bis $C_8$. Ganz besonders geeignet sind Acryl- oder Methacrylamidoacetaldehyd-di-n-pentylacetal.

Die Monomeren gemäß Formel I und II werden als Mischung der Saatdispersion zugesetzt, wobei die Monomerenmischung besteht aus der Polyhydroxy-Verbindung der Formel II in Mengen von 10 bis 90 Gew. %, vorzugweise 30 bis 70 Gew. % und aus der Acetalverbindung nach Formel I in Mengen von 90 bis 10 Gew. %, vorzugsweise 70 bis 30 Gew. % bestehen.

Dem Monomerengemisch können bis zu 30 Gew. %, bezogen auf die Gesamtmischung, Styrol, Vinylnaphthalin oder Vinyltoluol zugegeben werden. Außerdem kann das Monomerengemisch, gegebenenfalls zusätzlich, bis zu 30 Gew. %, bezogen auf die Gesamtmischung, Methacrylsäure, Acrylsäure oder Crotonsäure enthalten.

Vorteilhafterweise setzt man der aus den Monomeren bestehenden Mischung bis zu 20 Gew. %, bezogen auf die Monomerenmischung, Dimethylformamid oder andere geeignete Substanzen, die die Viskosität herabsetzen, zu.

Der Saatdispersion wird das Monomerengemisch zugegeben in Mengen von 90 bis 5 Gew. %, vorzugsweise 40 bis 10 Gew. %, bezogen auf die Gesamtmenge von Saatdispersion und Monomerenmischung.

Die Saatpolymerisation kann an sich nach üblichen Verfahren durchgeführt werden. Bevorzugte Ausführungsform des erfindungsgemäßen Verfahrens ist jedoch das Dosierverfahren, bei dem die Monomerenmischung unter ständigem Rühren der Suspension der Latexkerne bei Polymerisationsbedingungen, d. h. bei einer Temperatur von + 10° bis + 120° C, vorzugsweise + 50° bis + 90° C, zugetropft wird.

Anschließend wird das Polymerisat von überschüssigen Monomeren, Resten von Initiator und Emulgator nach bekannten Verfahren befreit. Vorteilhafterweise unterwirft man das Polymerisat einer Dialyse, zum Beispiel gegen $NaHCO_3$-Puffer (0,01 bis 0,05 Gew. %).

Zur Herstellung der erfindungsgemäßen, biologisch aktiven Dispersionspolymere, nachfolgend auch als Latexkonjugat bezeichnet, wird eine Suspension der oben beschriebenen saatpolymerisierten Latexpartikel auf einen pH-Wert unter 5, vorzugsweise unter 3, eingestellt und mit dem zu bindenden immunologisch

aktiven Material, wie zum Beispiel Antikörper, Haptene oder Antigene, inkubiert. Die labilen Bindungen zwischen einer Aminogruppe des Proteins und dem freigesetzten Aldehyd auf dem erfindungsgemäßen Latexpartikel werden nach bekannten Verfahren reduziert. Bevorzugt wird eine Lösung von Natriumcyanborhydrid in neutralem Puffer hierfür verwendet. Man trennt eventuell ungebundenes immunologisch aktives Material oder andere Verunreinigungen aus dem Reaktionsansatz ab. Dies geschieht zweckmäßigerweise durch Zentrifugieren oder Waschen auf geeigneten Membranen.

Die erfindungsgemäßen, saatpolymerisierten Dispersionspolymere zeichnen sich durch besondere Stabilität aus. Sie eignen sich zur Herstellung besonders empfindlicher Reagenzien, während bekannte Dispersionen, insbesondere solche mit hoher Nachweisempfindlichkeit, dazu neigen, schon nach relativ kurzer Zeit unspezifisch zu agglutinieren. Dies führt bei nephelometrischen oder turbidimetrischen Messungen zu einem Anstieg des Streulicht- beziehungsweise Extinktionssignals. Die Signale für die Messung einer Referenzkurve werden schon nach mehrstündiger Lagerung des Reagenzes so hoch, daß eine Auswertung nicht mehr möglich ist. Eine derartige Veränderung der Referenzkurve, aufgenommen mit einem Reagenz nach dem Stand der Technik bei unterschiedlichen IgE-Konzentrationen, zeigt Abbildung 1. Abbildung 3 zeigt die entsprechende Messung, durchgeführt jedoch mit dem erfindungsgemäßen Reagenz, das nach Beispiel 3 hergestellt ist. Es zeigt sich deutlich, daß das erfindungsgemäße Reagenz eine sehr viel niedrigere Eigenagglutination aufweist und seine Verwendung zu einem breiten, dynamischen Meßbereich führt.

Die Stabilität des erfindungsgemäßen Latexreagenzes zeigt sich auch dadurch, daß es einen bedeutend niedrigeren Reagenzblindwert aufweist als das Reagenz hergestellt nach dem Stand der Technik (vgl. Abbildung 3 und Abbildung 1). Sowohl bei mehrmonatiger (bis mindestens sechs Monate) Lagerung des Reagenzes bei + 4° bis + 8° C als auch bei mehrtägiger (bis mindestens 14 Tage) thermischer Belastung des lyophilisierten Reagenzes bei + 37° C erhält man nahezu unveränderte Ergebnisse für eine Referenzkurve gemessen mit unterschiedlichen Konzentrationen von IgE.

Die Nachteile herkömmlicher Reagenzien kommen auch dadurch zum Ausdruck, daß die mit ihnen durchgeführten nephelometrischen oder turbidimetrischen Messungen zu Ergebnissen führen, die mit denen eines Enzymimmunoassays nicht gut übereinstimmen. Abbildung 2 zeigt die Ergebnisse von nephelometrischen Messungen nach Beispiel 5 nach Stand der Technik, aber unter Verwendung der Latices für Serumproben mit einem IgE-Reagenz im Vergleich mit Ergebnissen von Enzymimmunoassays. Die Ergebnisse der gleichen nephelometrischen Messungen, jedoch durchgeführt mit einem IgE-Reagenz unter Verwendung der erfindungsgemäßen Dispersionspolymere gemäß Beispiel 1 und 3 zeigen Abbildung 4 und Abbildung 5. Wie ersichtlich wird eine hervorragende Übereinstimmung mit den Ergebnissen des Enzymimmunoassays erzielt.

Auch für unterschiedliche Patientenseren, die kein eigenes IgE enthalten, aber mit gleichen Mengen von aufgereinigtem IgE aufgestockt worden sind, erhält man mit den Reagenzien nach dem Stand der Technik je nach Serummatrix unterschiedliche Werte. Auch in dieser Hinsicht führen die erfindungsgemäßen Dispersionspolymere zu deutlichen Verbesserungen wie ein Vergleich der Meßwerte, die in Tabelle 1 zusammengestellt sind, mit den Meßwerten gemäß Tabelle 2 ergibt.

Die erfindungsgemäßen Dispersionspolymere sind einfach herzustellen und schonend mit empfindlichen immunologisch aktiven Materialien zu einem diagnostischen Reagenz zu verknüpfen. Die erfindungsgemäßen Dispersionspolymere und ihre daraus hergestellten biologisch aktiven Dispersionspolymere die Latexkonjugate und -reagenzien, sind stabil im Vergleich zu jenen des Standes der Technik. Sie sind unempfindlich gegen Störung durch Matrixeffekte, mit ihnen durchgeführte nephelometrische oder turbidimetrische Messungen führen zu den Ergebnissen, die auch mit einem Enzymimmunoassay erzielt werden. Im Vergleich zum Stand der Technik erhält man wesentlich weniger falschpositive oder falschnegative Ergebnisse. Bei Aufstockung IgE-freier Seren mit aufgereinigtem IgE findet man mit dem erfindungsgemäßen Reagenz Werte, die wenig schwanken und nahe dem theoretischen Wert liegen. Die geringere Anfälligkeit gegen Störeffekte zeigt sich auch in dem niedrigeren Wert des Variationskoeffizienten (vgl. Tabellen 1, 2). Die biologisch aktiven Dispersionspolymere können zum diagnostischen Nachweis von Antigenen, Antikörpern, oder Haptenen verwendet werden. Die Latexkonjugate können in allen diagnostischen Verfahren eingesetzt werden, die Teilchengrößenveränderungen messen, beispielsweise in qualitativen und semiquantitativen Bestimmungen von Substanzen mit Hilfe von visuellen Latexagglutinationstesten sowie zu nephelometrischen oder turbidimetrischen Bestimmungen, z.B. von Spurenproteinen im direkten oder kompetitiven Agglutinationstest oder im Latex-Hapten-Inhibitionstest, und für Partikelzählverfahren.

Beispiele

1 a) Synthese von N-(2,3-Dihydroxypropyl)-Methacrylamid

4,56 g 3-Amino-1,2-Propandiol (0,05 mol) wurden in 30 ml Dimethylformamid (wasserfrei) gelöst. Diese Lösung sowie 13,8 g $K_2CO_3$ wurden in einem 100 ml Dreihalskolben mit Tropftrichter und Gasein- sowie Gasauslaßrohr gegeben. Die Mischung wurde im Eisbad auf 0° C abgekühlt. Unter mäßigem Rühren und leichtem Durchperlen von Stickstoff wurden während 30 Minuten 6,18 ml Methacrylsäurechlorid (0,06 mol), in 30 ml Dimethylformamid gelöst, zugetropft. Nach einer weiteren Stunde Rühren unter Eiskühlung ließ man die Mischung sich auf Raumtemperatur erwärmen und rührte weitere 30 Minuten. Der Reaktionsansatz wurde durch ein Faltenfilter filtriert und der Rückstand verworfen. Das Filtrat wurde am Rotationsverdampfer eingeengt bis ein viskoses Öl entstand. Dieses Öl wurde in 30 ml Methanol gelöst, ein zweites Mal filtriert und das Filtrat wiederum am Rotationsverdampfer eingeengt. Die Reste des Lösungsmittels wurden im Hochvakuum entfernt. Die Ausbeute betrug 8,52 g.

b) Polymerisation von N-(2,3-Dihydroxypropyl)-Methacrylamid (NDPM) auf Polystyrolkerne

In ein zylindrisches Glasgefäß, ausgestattet mit Gasein- und Gasauslaßrohr sowie einem Magnetrührstab wurden 22,3 ml einer Polystyrol-Latex-Dispersion mit einem Feststoffgehalt von 17,9 Gew. % sowie 57 ml destilliertes Wasser und 50 mg Natriumdodecylsulfat gegeben und durch Rühren gelöst. Durch mehrfaches Evakuieren und Füllen mit Stickstoff wurde das Polymerisationsgefäß sauerstoffrei gemacht. Die Latex-Detergenzmischung wurde unter ständigem Rühren auf + 70° C in einem Wasserbad erhitzt. Man gab 1 ml einer Kaliumperoxydisulfatlösung (16 mg/ml in destilliertem Wasser) hinzu.

Es wurde eine Monomerenmischung, hergestellt aus 0,2 ml Styrol, 0,4 ml Methacrylamidoacetaldehyd-di-n-pentylacetal, 0,025 ml Methacrylsäure und 0,4 ml des im Beispiel 1 a) erhaltenen N-(2,3-Dihydroxypropyl)-Methacrylamid (NDPM) sowie zur besseren Löslichkeit dieser Monomere 0,2 ml Dimethylformamid.

Unter starkem Rühren der Polystyrol-Latex-Suspension wurde zu dieser die Mischung der Monomere während 60 Minuten langsam hinzugetropft. Die Temperatur des Polymerisationsansatzes wurde auf 70° C gehalten. Nach dem Zutropfen der Monomerenmischung wurde weitere 4 Stunden bei der genannten Temperatur gerührt. Die Polymerisation war damit beendet und die Dispersion wurde auf Raumtemperatur abgekühlt und mittels Faltenfilter filtriert. Man erhielt 73 ml einer Latex-Suspension. Diese wurde anschließend 17 Stunden lang gegen eine $NaHCO_3$-Pufferlösung (0,25 g/l, pH 8-8,2) dialysiert. Man erhielt 80 ml einer Latex-Dispersion mit einem Feststoffgehalt von 4,7 Gew. %.

2 a) Synthese von N-(2-Hydroxypropyl)-Methacrylamid

3,75 g 1-Amino-2-Propanol (0,05 mol) wurden zusammen mit 30 ml Acetonitril (wasserfrei) und 13,79 g $K_2CO_3$ (0,1 mol) in einem Dreihalskolben mit Tropftrichter und Gasein- sowie Gasauslaßrohr vorgelegt und im Eisbad auf 0° C abgekühlt. Unter stetem Rühren und leichtem Durchleiten von Stickstoff wurden während 30 Minuten 6,18 g Methacrylsäurechlorid (0,06 mol), gelöst in 30 ml Acetonitril, zugetropft. Nach weiteren 60 Minuten Rühren unter Eiskühlung ließ man die Mischung sich auf Raumtemperatur erwärmen und rührte weitere 30 Minuten. Der Ansatz wurde durch eine Glasfritte filtriert und der Niederschlag verworfen. Das Filtrat wurde am Rotationsverdampfer eingeengt, bis ein viskoses Öl entstand. Dieses wurde in 30 ml Methanol gelöst und vom eventuell auftretenden Präzipitat abgetrennt. Das Filtrat wurde wiederum am Rotationsverdampfer eingeengt und die Reste des Lösungsmittels wurden im Hochvakuum entfernt. Die Ausbeute betrug 6,66 g.

b) Polymerisation von N-(2-Hydroxypropyl)-Methacrylamid auf Polystyrolkerne

Die Polymeristion erfolgte ähnlich wie im Beispiel 1 b) beschrieben. Man stellte eine Mischung von 24,2 ml Polystyrol-Latex mit einem Feststoffgehalt von 16,5 Gew. % und 54,8 ml destilliertes Wasser sowie 50 mg Natriumdodecylsulfat her. Diese wurde in das Polymerisationsgefäß gegeben und der Sauerstoff entfernt. Weiterhin wurden 1 ml einer Kaliumperoxodisulfatlösung (16 mg/ml in destilliertem Wasser) hinzugegeben und der Ansatz auf + 70° C erhitzt. Man stellte eine Mischung aus 0,4 ml Styrol, 0,4 ml Methacrylamidoacetaldehyd-di-n-pentylacetal, 0,025 ml Methacrylsäure und 0,4 ml N-(2-Hydroxypropyl)-Methacrylamid her.

Die Monomerenmischung wurde zur kräftig gerührten Polystyrol-Latex-Dispersion bei + 70° C während 60 Minuten langsam hinzugetropft. Anschließend wurde weitere 4 Stunden bei gleicher Temperatur weitergerührt. Nach Abkühlung auf Raumtemperatur und Filtration durch ein Faltenfilter erhielt man 74 ml des Polymerisats. Man dialysierte anschließend ca. 20 Stunden gegen $NaHCO_3$-Puffer (0,25 g/1, pH 8-8,2). Man erhielt 79 ml einer Latex-Suspension mit einem Feststoffgehalt von 5,4 Gew. %.

3. Polymerisation von 2-Hydroxypropylmethacrylat (HPM) auf Polystyrolkerne

Die Polymerisation erfolgte ähnlich wie im Beispiel 1 b) beschrieben. Man stellte eine Mischung von 22,4 ml Polystyrol-Latex mit einem Feststoffgehalt von 17,9 Gew. % und 56,7 ml destilliertem Wasser sowie 50 mg Natriumdodecylsulfat her. Diese wurde in das Polymerisationsgefäß gegeben und der Sauerstoff entfernt. Weiterhin wurden 1 ml einer Kaliumperoxid-Disulfatlösung (16 mg/ml in destilliertem Wasser) hinzugegeben und der Ansatz auf + 70° C erhitzt. Man stellte eine Mischung aus 0,4 ml Styrol, 0,4 ml Methacrylamidoacetaldehyd-di-n-pentylacetal, 0,025 ml Methacrylsäure und 0,2 ml 2-Hydroxypropyl-methacrylat (HPM) her. Die Monomerenmischung wurde zur kräftig gerührten Polystyrol-Latex-Suspension bei + 70° C während 60 Minuten langsam hinzugetropft. Anschließend wurde weitere 4 Stunden bei gleicher Temperatur weitergerührt.

Nach Abkühlung auf Raumtemperatur und Filtration durch ein Faltenfilter erhielt man 73 ml des Polymerisats. Man dialysierte anschließend ca. 20 Stunden gegen $NaHCO_3$-Puffer (0,25 g/l, pH 8-8,2). Man erhielt 87 ml einer Latex-Dispersion mit einem Feststoffgehalt von 5,1 %.

4. Bindung von Anti-IgE-Antikörpern an ein erfindungsgemäßes Polymerisat

An ein Polymerisat unter Verwendung von N-(2,3-Dihydroxypropyl)-methacrylamid hergestellt nach Beispiel 1 oder unter Verwendung von N-(2,3-Hydroxypropyl)-methacrylamid hergestellt nach Beispiel 2 oder unter Verwendung von 2-Hydroxypropyl-methacrylat hergestellt nach Beispiel 3, wurden Anti-IgE-Antikörper gebunden.

Das jeweils eingesetzte Polymerisat wurde mit destilliertem Wasser auf einen Feststoffgehalt von 4 Gew. % verdünnt. Ein Antiserum, gewonnen durch Immunisierung von Kaninchen mit aufgereinigtem IgE, wurde nach bekannten Verfahren affinitätschromatographisch aufgereinigt. Es wurde anschließend aufkonzentriert bis ein Proteingehalt von 10 mg/ml erreicht war.

3,4 ml des obengenannten Polymerisates wurden mit 0,34 ml der Anti-IgE-Antikörper-Lösung gemischt. Dann wurden 0,17 ml einer 20 %igen wässrigen Lösung von Eikosaoxyethylen-sorbitan-laurylsäurester (®Tween 20) hinzugegeben und das Ganze nochmals gemischt. Hierzu gaben wir 0,05 ml 1N HCl, so daß ein pH-Wert von ca. 2 erreicht wurde. Nach einer Inkubationszeit von 30 Minuten bei Raumtemperatur setzten wir 0,85 ml gesättigte wässrige Natriumhydrogenphosphat-Lösung (pH 6,5) und 0,85 ml wässrige Natriumcyanborhydrid-Lösung (25 mg/ml) hinzu und mischten gut durch. Anschließend erfolgte eine Inkubation von einer Stunde bei Raumtemperatur.

Dieser Beladungsansatz wurde sodann 30 Minuten mit ca. 50.000 g zentrifugiert (Beckman Zentrifuge, 20.000 UpM). Der Überstand wurde verworfen. Der Rückstand wurde in 5 ml eines Glycin-NaCl-Puffers (0,1 Mol Glycin, 0,17 Mol NaCl, 0,5 % Eikosaoxyethylen-sorbitan-laurylsäureester( ®Tween 20), pH 8,2) resuspendiert. Anschließend erfolgte eine Ultraschall-Behandlung (Bronson Sonyfier B 15) für 2 Sekunden. Das so redispergierte Reagenz wurde im Volumenverhältnis 1:80 mit dem zuvor genannten Glycin-NaCl-Puffer verdünnt.

5. Messung von IgE-Konzentrationen in Serumproben

Das nach Beispiel 4 durch Bindung von Anti-IgE-Antikörpern an erfindungsgemäßen Latexpräparationen hergestellte Reagenz zur Bestimmung von IgE wurde zur Messung von IgE in Patientenseren eingesetzt. Als Standard wurde die oberste Standardabfüllung mit der höchsten IgE-Konzentration des Enzygnost-Testes (Fa. Behringwerke AG) verwendet. Dieser IgE-Standard enthielt laut Packungsbeilage 1.000 IU/ml. Der Standard wurde in einem IgE-freien Serumpool stufenweise auf jeweils das doppelte Volumen weiterverdünnt. Man erhielt so eine Standardreihe mit abnehmenden IgE-Konzentrationen. Die Standardsera wie auch zu bestimmende Patientensera wurden 1:5 in einem Glycin-Kochsalz-Puffer (0,1 Mol Glycin, 0,17 Mol NaCl, pH 8,2) verdünnt. Zur Messung wurden 20 µl Patientenserum-Verdünnung beziehungsweise Standardserum-Verdünnung mit 150 µl eines Reaktionspuffers (0,1 Mol Glycin, 0,17 Mol NaCl, 4 % Polyethylenglykol (PEG) 6000, 0,5 % ®Tween 20, pH 8,2) in BLN-Küvetten (Fa. Behringwerke AG) gemischt und 30 Minuten bei Raumtemperatur inkubiert. Die Küvetten wurden dann im Lasernephelometer (Fa. Behringwerke AG) gemessen. Die Referenzkurve für die Messung der Standardseren wurde auf halblogarithmischem Papier gezeichnet und daran wurden die Meßwerte für die Patientenseren ausgewertet. Eine typische Referenzkurve ist in Abbildung 3 dargestellt.

Tabelle 1

Meßwerte im nephelometrischen Test, Stand der Technik

| Serum Nr. | Gehalt IgE in IU/ml |
|---|---|
| 1 | 441 |
| 2 | 399 |
| 3 | 556 |
| 4 | 416 |
| 5 | 431 |
| 6 | 425 |
| 7 | 454 |
| 8 | 398 |
| 9 | 422 |
| 10 | 356 |
| 11 | 315 |
| 12 | 382 |

Mittelwert = 416 IU/ml = 80 % Wiederfindung
Sollwert = 520 IU/ml
Variationskoeffizient = 14 %

Meßwerte im nephelometrischen Test nach Beispiel 5 für Serumproben, die mit jeweils 520 IU/ml IgE aufgestockt worden sind. Die Messungen erfolgten mit einem Reagenz, hergestellt nach dem Stand der Technik.

Tabelle 2

Meßwerte im nephelometrischen Test, erfindungsgemäßes Reagenz

| Serum Nr. | Gehalt IgE in IU/ml |
|---|---|
| 1 | 435 |
| 2 | 516 |
| 3 | 609 |
| 4 | 537 |
| 5 | 526 |
| 6 | 571 |
| 7 | 608 |
| 8 | 559 |
| 9 | 589 |
| 10 | 542 |
| 11 | 559 |
| 12 | 450 |

Mittelwert = 541 IU/ml = 104 % Wiederfindung
Sollwert = 520 IU/ml
Variationskoeffizient = 10 %

Meßwerte im nephelometrischen Test nach Beispiel 5 für Serumproben, die mit jeweils 520 IU/ml IgE aufgestockt worden sind. Die Messungen erfolgten mit einem erfindungsgemäßen Reagenz, hergestellt nach Beispiel 3.

**Patentansprüche**

1. Dispersionspolymere, bestehend aus Latexteilchen in einem wässrigen Medium, auf deren Oberfläche sich ein Copolymerisat befindet, das Monomeren mit endständigen Acetalen der Formel I

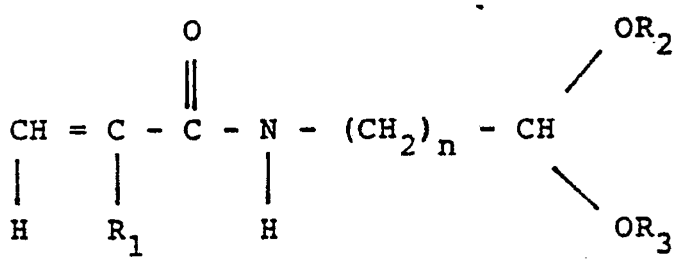

I

worin

n = 1 - 6;
$R_1$ = H, $CH_3$ und
$R_2$ und $R_3$ gleich oder unterschiedlich sind mit
$R_2$, $R_3$ = $-(CH_2)_m-CH_3$ m = 0 - 7 oder

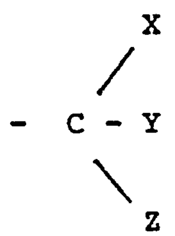

, wobei X, Y, Z = $(CH_2)_p-CH_3$ und p = 1 - 3 sind, mit X, Y, Z gleich oder unterschiedlich

und Monomeren der Formel II

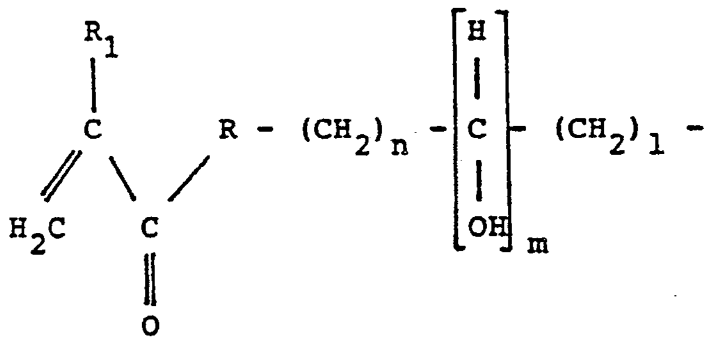

II

worin

R = O,NH; n = 1 - 3; m = 1 - 4; 1 = 0 - 4
$R_1$ = H, $CH_3$,

enthält.

2. Dispersionspolymere nach Anspruch 1, wobei als Monomere der Formel II Mono- oder Dihydroxyalkylacryl- oder methacrylverbindungen eingesetzt sind.

3. Dispersionspolymere nach Anspruch 1, wobei als Monomer der Formel II eingesetzt ist wenigstens eine der Verbindungen N-2,3-Dihydroxypropylmethacrylamid, N-2-Hydroxypropylmethacrylamid, 2-Hydroxypropylmethacrylat oder N-2,3-Dihydroxypropylacrylamid, N-2-Hydroxypropylacrylamid oder 2-Hydroxypropylacrylat.

4. Dispersionspolymere nach Anspruch 1, wobei als Monomere der Formel I Acryl- oder Methacrylamidoalkylaldehyd-di-alkylacetal mit Alkyl = $C_2$ bis $C_8$ eingesetzt sind.

5. Dispersionspolymere nach Anspruch 1, worin als Monomer der Formel I Methacrylamidoacetaldehyd-di-n-pentylacetal verwendet wird.

6. Verfahren zur Herstellung von Dispersionspolymeren nach Anspruch 1, dadurch gekennzeichnet, daß zu einer Saatdispersion, bestehend aus Latex teilchen in einem wässrigen Medium mit einem Partikeldurchmesser von 0,02 bis 2 $\mu m$ Emulgator und Radikalstarter zugesetzt werden und sodann unter Rühren bei einer Temperatur von + 10° bis + 120° C eine Monomerenmischung zugetropft wird, die eine Verbindung der Formel II und einer Verbindung der Formel I enthält.

7. Verfahren nach Anspruch 6, dadurch gekennzeichnet, daß die Monomerenmischung als Verbindung der Formel II enthält Mono- oder Dihydroxyalkylacryl- oder methacrylverbindungen und als Verbindung der Formel I Acryl- oder Methacrylamidoalkylaldehyd-di-alkylacetal mit Alkyl = $C_2$ bis $C_8$ eingesetzt wird.

8. Verfahren nach Anspruch 6, dadurch gekennzeichnet, daß die Monomerenmischung als Verbindung der Formel II enthält N-2,3-Dihydroxypropylmethacrylamid, N-2-Hydroxypropylmethacrylamid, 2-Hydroxypropylmethacrylat oder die entsprechenden Acrylverbindungen und als Verbindung der Formel I eingesetzt wird Acryl- oder Methacrylamidoalkylacetaldehyd-di-n-pentylacetal.

9. Verfahren gemäß Anspruch 6, dadurch gekennzeichnet, daß die Monomerenmischung zusätzlich bis zu 30 Gew. %, bezogen auf die Gesamtmischung, Styrol, Vinylnaphthalin oder Vinyltoluol enthält.

10. Verfahren nach Anspruch 6, dadurch gekennzeichnet, daß das Monomerengemisch zusätzlich bis zu 30 Gew. %, bezogen auf die Gesamtmischung, Methacrylsäure, Acrylsäure oder Crotonsäure enthält.

11. Verfahren nach Anspruch 6, dadurch gekennzeichnet, daß die Monomerenmischung zusätzlich zu Monomeren der Formel I und II sowie Verbindungen aus der Gruppe, Styrol, Vinylnaphthalin oder Vinyltoluol und einer der Verbindungen Methacrylsäure, Acrylsäure oder Crotonsäure zusätzlich noch bis zu 20 Gew. % bezogen auf die Gesamtmischung, Dimethylformamid enthält.

12. Biologisch aktive Dispersionspolymere, bestehend aus Dispersionspolymeren gemäß Anspruch 1 und darin gebundene Antikörper, Antigene oder Haptene.

13. Verwendung eines biologisch aktiven Dispersionspolymeren nach Anspruch 12 zum diagnostischen Nachweis von Antigenen, Antikörpern oder Haptenen.

14. Verwendung eines biologisch aktiven Dispersionspolymeren nach Anspruch 12 in nephelometrischem und turbidimetrischem Verfahren sowie für Partikelzählverfahren.

**Patentansprüche für folgenden Vertragsstaat: ES**

1. Verfahren zur Herstellung von Dispersionspolymeren, bestehend aus Latexteilchen, auf deren Oberfläche sich ein Copolymerisat befindet, das Monomeren der Formel I

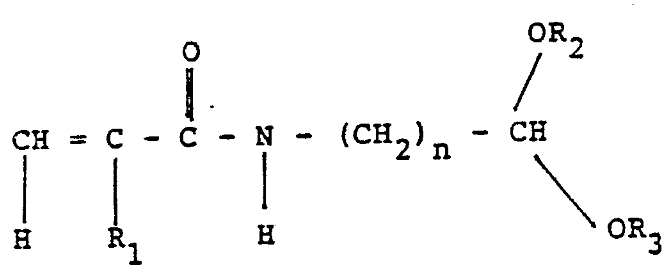

I

worin

| | |
|---|---|
| n = | 1 - 6; |
| $R_1$ = | H, $CH_3$ und |
| $R_2$ und $R_3$ | gleich oder unterschiedlich sind mit |
| $R_2$, $R_3$ = | $-(CH_2)_m-CH_3$ m = 0 - 7 oder |

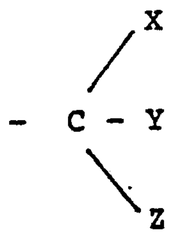

wobei X, Y, Z = $(CH_2)_p$-$CH_3$ und
p = 1 - 3 sind,
mit X, Y, Z gleich oder unterschiedlich

und Monomeren der Formel II

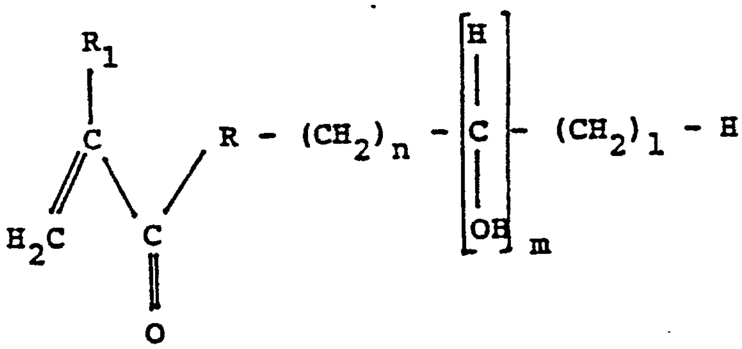

II

worin
R = O,NH; n = 1 - 3; m = 1 - 4; 1 = 0 - 4
R = H, $CH_3$
enthält,
dadurch gekennzeichnet, daß zu einer Saatdispersion, bestehend aus Latex teilchen in einem wässrigen Medium mit einem Partikeldurchmesser von 0,02 bis 2 µm Emulgator und Radikalstarter zugesetzt werden und sodann unter Rühren bei einer Temperatur von + 10° bis + 120° C eine Monomerenmischung zugetropft wird, die eine Verbindung der Formel II und einer Verbindung der Formel I enthält.

2. Verfahren nach Anspruch 1, dadurch gekennzeichnet, daß die Monomerenmischung als Verbindung der Formel II enthält Mono- oder Dihydroxyalkylacryl- oder methacrylverbindungen und als Verbindung der Formel I Acryl- oder Methacrylamidoalkylaldehyd-di-alkylacetal mit Alkyl = $C_2$ bis $C_8$ eingesetzt wird.

3. Verfahren nach Anspruch 1, dadurch gekennzeichnet, daß die Monomerenmischung als Verbindung der Formel II enthält N-2,3-Dihydroxypropylmethacrylamid, N-2-Hydroxypropylmethacrylamid, 2-Hydroxypropylmethacrylat oder die entsprechenden Acrylverbindungen und als Verbindung der Formel I eingesetzt wird Acryl- oder Methacrylamidoalkylacetaldehyd-di-n-pentylacetal.

4. Verfahren gemäß Anspruch 1, dadurch gekennzeichnet, daß die Monomerenmischung zusätzlich bis zu 30 Gew. %, bezogen auf die Gesamtmischung, Styrol, Vinylnaphthalin oder Vinyltoluol enthält.

5. Verfahren nach Anspruch 1, dadurch gekennzeichnet, daß das Monomerengemisch zusätzlich bis zu 30 Gew. %, bezogen auf die Gesamtmischung, Methacrylsäure, Acrylsäure oder Crotonsäure enthält.

6. Verfahren nach Anspruch 1, dadurch gekennzeichnet, daß die Monomerenmischung zusätzlich zu Monomeren der Formel I und II sowie Verbindungen aus der Gruppe, Styrol, Vinylnaphthalin oder Vinyltoluol und einer der Verbindungen Methacrylsäure, Acrylsäure oder Crotonsäure zusätzlich noch bis zu 20 Gew. % bezogen auf die Gesamtmischung, Dimethylformamid enthält.

**Claims**

1. Dispersion polymers, comprising latex particles in an aqueous medium, on the surface of which particles a copolymer is located which contains monomers having terminal acetals of the formula I

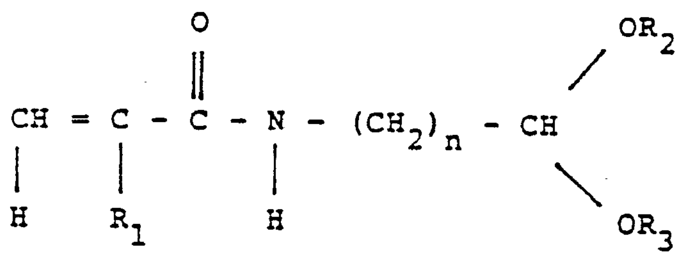

I

in which

- n denotes 1 - 6;
- $R_1$ denotes H or $CH_3$, and
- $R_2$ and $R_3$ are identical or different and denote $-(CH_2)_m-CH_3$, and
- m denotes 0 - 7, or

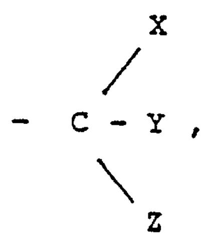

in which
X, Y and Z denote $(CH_2)_p-CH_3$ and
p denotes 1 - 3, where
X, Y and Z are identical or different,
and monomers of the formula II

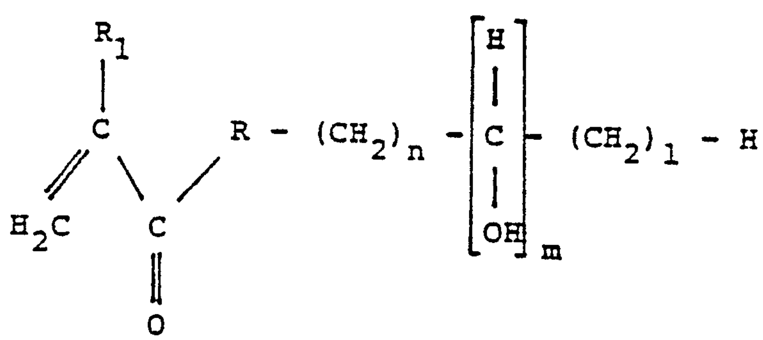

II

in which

- R denotes O or NH;
- n denotes 1 - 3;
- m denotes 1 - 4;
- 1 denotes 0 - 4; and
- $R_1$ denotes H or $CH_3$.

**2.** Dispersion polymers as claimed in claim 1, mono- or dihydroxyalkylacrylic or methacrylic compounds being employed as monomers of the formula II.

**3.** Dispersion polymers as claimed in claim 1, at least one of the compounds N-2,3-dihydroxypropylmethacrylamide, N-2-hydroxypropylmethacrylamide, 2-hydroxypropyl methacrylate or N-2,3-dihydroxypropylacrylamide, N-2-hydroxypropylacrylamide or 2-hydroxypropylacrylate being employed as monomer of the formula II.

4. Dispersion polymers as claimed in claim 1, acrylamidoalkyl- or methacrylamidoalkyl-aldehyde di-alkyl acetal where alkyl denotes $C_2$ to $C_8$ being employed as monomers of the formula I.

5. Dispersion polymers as claimed in claim 1, in which methacrylamido-acetaldehyde di-n-pentyl acetal is used as monomer of the formula I.

6. A process for the preparation of dispersion polymers as claimed in claim 1, which comprises adding emulsifier and free-radical initiator to a seed dispersion, comprising latex particles having a particle diameter of 0.02 to 2 µm in an aqueous medium, and then adding a mixture of monomers, containing a compound of the formula II and a compound of the formula I, dropwise, with stirring, at a temperature of +10°C to +120°C.

7. The process as claimed in claim 6, wherein the mixture of monomers contains, as compound of the formula II, mono- or dihydroxyalkylacrylic or methacrylic compounds, and acrylamidoalkyl or methacrylamidoalkyl aldehyde dialkyl acetal where alkyl denotes $C_2$ to $C_8$ is employed as compound of the formula I.

8. The process as claimed in claim 6, wherein the mixture of monomers contains, as compound of the formula II, N-2,3-dihydroxypropylmethacrylamide, N-2-hydroxypropylmethacrylamide, 2-hydroxypropyl methacrylate or the corresponding acrylic compounds, and acrylamidoalkyl or methacrylamidoalkyl acetaldehyde di-n-pentyl acetal is employed as compound of the formula I.

9. The process as claimed in claim 6, wherein the mixture of monomers additionally contains up to 30% by weight, based on the total mixture, of styrene, vinylnaphthalene or vinyltoluene.

10. The process as claimed in claim 6, wherein the mixture of monomers additionally contains up to 30% by weight, based on the total mixture, of methacrylic acid, acrylic acid or crotonic acid.

11. The process as claimed in claim 6, wherein the mixture of monomers contains, in addition to monomers of the formulae I and II, compounds from the group comprising styrene, vinylnaphthalene or vinyltoluene, and one of the compounds methacrylic acid, acrylic acid or crotonic acid, and in addition up to 20% by weight, based on the total mixture, of dimethylformamide.

12. Biologically active dispersion polymers, comprising dispersion polymers as claimed in claim 1 and antibodies, antigens or haptens bonded therein.

13. The use of a biologically active dispersion polymer as claimed in claim 12 for the diagnostic detection of antigens, antibodies and haptens.

14. The use of a biologically active dispersion polymer as claimed in claim 12 in the nephelometric and turbidimetric method and also for particle counting methods.

**Claims for the following Contracting State: ES**

1. A process for the preparation of dispersion polymers, comprising latex particles and on whose surface a copolymer is located which contains monomers of the formula I

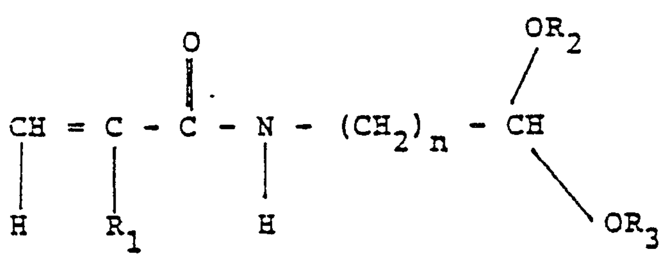

I

in which

n denotes 1 - 6;
$R_1$ denotes H or $CH_3$, and
$R_2$ and $R_3$ are identical or different and denote $-(CH_2)_m-CH_3$, and
m denotes 0 - 7, or

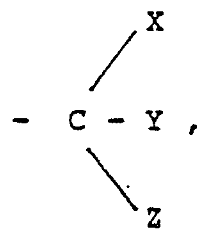

in which
X, Y and Z denote $(CH_2)_p-CH_3$ and
p denotes 1 - 3, where
X, Y and Z are identical or different,
and monomers of the formula II

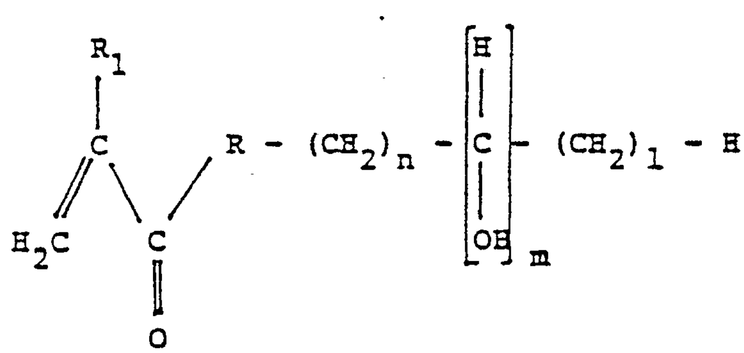
II

in which
R denotes O or NH;
n denotes 1 - 3;
m denotes 1 - 4;
1 denotes 0 - 4; and
$R_1$ denotes H or $CH_3$, which comprises adding emulsifier and free - radical initiator to a seed dispersion, comprising latex particles having a particle diameter of 0.02 to 2 µm in an aqueous medium, and then adding a mixture of monomers, containing a compound of the formula II and a compound of the formula I, dropwise, with stirring, at a temperature of +10° to +120° C.

**2.** The process as claimed in claim 1, wherein the mixture of monomers contains, as compound of the formula II, mono- or dihydroxyalkylacrylic or methacrylic compounds, and acrylamidoalkyl or methacrylamidoalkyl aldehyde dialkyl acetal where alkyl denotes $C_2$ to $C_8$ is employed as compound of the formula I.

**3.** The process as claimed in claim 1, wherein the mixture of monomers contains, as compound of the formula II, N-2,3-dihydroxypropylmethacrylamide, N-2-hydroxypropylmethacrylamide, 2-hydroxypropyl methacrylate or the corresponding acrylic compounds, and acrylamidoalkyl- or methacrylamidoalkyl acetaldehyde- di-n-pentyl acetal is employed as compound of the formula I.

**4.** The process as claimed in claim 1, wherein the mixture of monomers additionally contains up to 30% by weight, based on the total mixture, of styrene, vinylnaphthalene or vinyltoluene.

**5.** The process as claimed in claim 1, wherein the mixture of monomers additionally contains up to 30%

by weight, based on the total mixture, of methacrylic acid, acrylic acid or crotonic acid.

**6.** The process as claimed in claim 1, wherein the mixture of monomers contains, in addition to monomers of the formulae I and II, compounds from the group comprising styrene, vinylnaphthalene or vinyltoluene, and one of the compounds methacrylic acid, acrylic acid or crotonic acid, and in addition up to 20% by weight, based on the total mixture, of dimethylformamide.

**Revendications**

**1.** Polymères en dispersion, constitués par des particules de latex en milieu aqueux, à la surface desquelles se trouve un copolymérisat qui contient des monomères, avec des acétals en position terminale, de formule I :

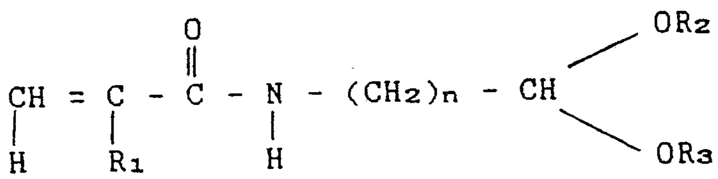

I

où

n = 1 à 6
$R_1$ = H, $CH_3$ et
$R_2$ et $R_3$ sont identiques ou différents avec
$R_2$, $R_3$ = $-(CH_2)_m-CH_3$, m = 0 à 7 ou

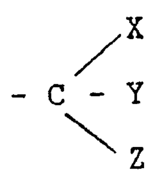

,

où

X, Y, Z = $(CH_2)_p-CH_3$ et
p = 1 à 3
avec X, Y, Z identiques ou différents
et des monomères de formule II:

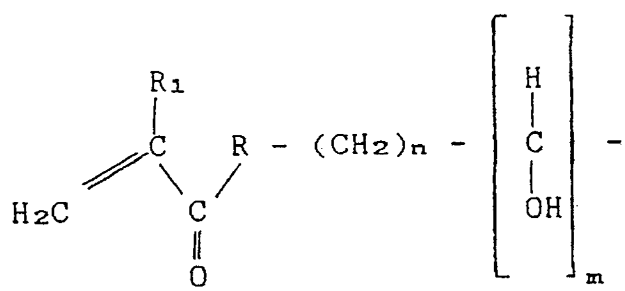

II

où

R = 0, NH; n = 1 à 3; m = 1 à 4; 1 = 0 à 4
$R_1$ = H, $CH_3$.

**2.** Polymères en dispersion selon la revendication 1, dans lesquels les monomères de formule II sont des composés méthacryliques ou acryliques, de mono- ou de dihydroxyalkyle.

3. Polymères en dispersion selon la revendication 1, dans lesquels le monomère de formule II est au moins un des composés choisis parmi le N-2,3-dihydroxypropylméthacrylamide, le N-2-hydroxypropylméthacrylamide, le méthacrylate de 2-hydroxypropyle ou le N-2,3-dihydroxypropylacrylamide, le N-2-hydroxypropylacrylamide ou l'acrylate de 2-hydroxypropyle.

4. Polymères en dispersion selon la revendication 1, dans lesquels les monomères de formule I sont des acryl- ou méthacrylamidoalkylaldéhyde-di-alkylacétals avec le groupe alkyle en $C_2$ à $C_8$.

5. Polymères en dispersion selon la revendication 1, dans lesquels le monomère de formule I est le méthacrylamidoacétaldéhyde-di-n-pentylacétal.

6. Procédé pour préparer des polymères en dispersion selon la revendication 1, caractérisé en ce que l'on ajoute, à une dispersion d'ensemencement, constituée de particules de latex ayant un diamètre de particules de 0,02 à 2 $\mu$m en milieu aqueux, un émulsifiant et un amorceur de radicaux libres, et on ajoute ensuite goutte-à-goutte, sous agitation, à une température comprise entre + 10 °C et +120 °C, un mélange de monomères contenant un composé de formule II et un composé de formule I.

7. Procédé selon la revendication 6, caractérisé en ce que le mélange de monomères contient, comme composé de formule II, des composés acryliques ou méthacryliques de mono- ou de dihydroxyalkyle et l'on utilise, comme composé de formule I, un acryl- ou méthacrylamidoalkylaldéhyde-di-alkylacétal, avec le groupe alkyle en $C_2$ à $C_8$.

8. Procédé selon la revendication 6, caractérisé en ce que le mélange de monomères contient, comme composé de formule II, le N-2,3-dihydroxypropylméthacrylamide, le N-2-hydroxypropylméthacrylamide, le méthacrylate de 2-hydroxypropyle ou les composés acryliques correspondants et on utilise, comme composé de formule I, l'acryl- ou le méthacrylamidoacétaldéhyde-di-n-pentylacétal.

9. Procédé selon la revendication 6, caractérisé en ce que le mélange de monomères contient de plus jusqu'à 30% en poids, par rapport au poids total du mélange, de styrène, de vinylnaphtalène ou de vinyltoluène.

10. Procédé selon la revendication 6, caractérisé en ce que le mélange de monomères contient de plus jusqu'à 30% en poids, par rapport au poids total du mélange, d'acide méthacrylique, d'acide acrylique ou d'acide crotonique.

11. Procédé selon la revendication 6, caractérisé en ce que le mélange de monomères contient encore, outre des monomères de formules I et II et des composés du groupe styrène, vinylnaphtalène ou vinyltoluène et l'un des composés acide méthacrylique ou acide crotonique, jusqu'à 20% en poids, par rapport au poids total du mélange, de diméthylformamide.

12. Polymères pour dispersion biologiquement actifs, constitués de polymères en dispersion selon la revendication 1 et d'anticorps, d'antigènes ou d'haptènes, liés à ceux-ci.

13. Utilisation d'un polymère en dispersion biologiquement actif selon la revendication 12 pour la mise en évidence diagnostique d'antigènes, d'anticorps ou d'haptènes.

14. Utilisation d'un polymère en dispersion biologiquement actif selon la revendication 12 dans un procédé néphélométrique ou turbidimétrique et dans un procédé de comptage de particules.

**Revendications pour l'Etat contractant suivant: ES**

1. Procédé pour préparer des polymères en dispersion constitués par des particules de latex en milieu aqueux, à la surface desquelles se trouve un copolymérisat qui contient des monomères, avec des acétals en position terminale, de formule I:

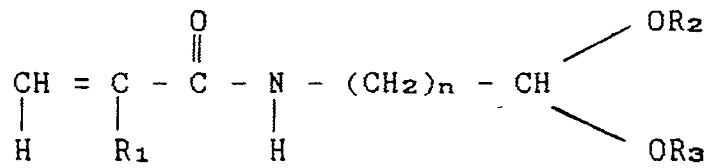

I

où

n = 1 à 6

$R_1$ = H, $CH_3$ et

$R_2$ et $R_3$ sont identiques ou différents avec

$R_2$, $R_3$ = - $(CH_2)_m$ - $CH_3$, m = 0 à 7 ou

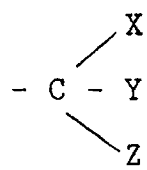

,

où

X, Y, Z = $(CH_2)_p$-$CH_3$ et

p = 1 à 3

avec X, Y, Z identiques ou différents

et des monomères de formule II:

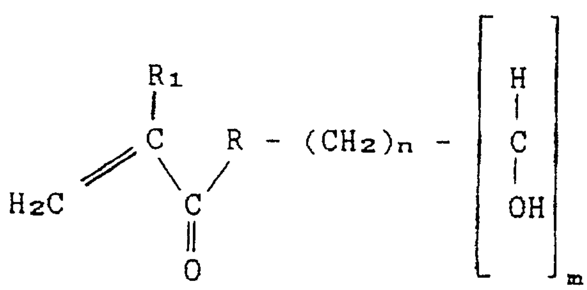

II

où

R = 0, NH; n = 1 à 3; m = 1 à 4; 1 = 0 à 4

$R_1$ = H, $CH_3$,

caractérisé en ce que l'on ajoute, à une dispersion d'ensemencement , constituée de particules de latex ayant un diamètre de particules de 0,02 à 2 µm en milieu aqueux, un émulsifiant et un amorceur de radicaux libres, et on ajoute ensuite goutte-à-goutte, sous agitation, à une température comprise entre + 10° C et +120° C, un mélange de monomères contenant un composé de formule II et un composé de formule I.

**2.** Procédé selon la revendication 1, caractérisé en ce que le mélange de monomères contient, comme composé de formule II, des composés acryliques ou méthacryliques de mono- ou de dihydroxyalkyle et l'on utilise, comme composé de formule I, un acryl- ou méthacrylamidoalkylaldéhyde-di-alkylacétal, avec le groupe alkyle en $C_2$ à $C_8$.

**3.** Procédé selon la revendication 1; caractérisé en ce que le mélange de monomères contient, comme composé de formule II, le N-2,3-dihydroxypropylméthacrylamide, le N-2-hydroxypropylméthacrylamide, le méthacrylate de 2-hydroxypropyle ou les composés acryliques correspondants et l'on utilise, comme composé de formule I, l'acryl- ou le méthacrylamidoacétaldéhyde-di-n-pentylacétal.

4. Procédé selon la revendication 1, caractérisé en ce que le mélange de monomères contient de plus jusqu'à 30% en poids, par rapport au poids total du mélange, de styrène, de vinylnaphtalène ou de vinyltoluène.

5. Procédé selon la revendication 1, caractérisé en ce que le mélange de monomères contient de plus jusqu'à 30% en poids, par rapport au poids total du mélange, d'acide méthacrylique, d'acide acrylique ou d'acide crotonique.

6. Procédé selon la revendication 1, caractérisé en ce que le mélange de monomères contient encore, outre des monomères de formules I et II et des composés du groupe styrène, vinylnaphtalène ou vinyltoluène et l'un des composés acide méthacrylique, acide acrylique ou acide crotonique, jusqu'à 20% en poids, par rapport au poids total du mélange, de diméthylformamide.

FIG.1

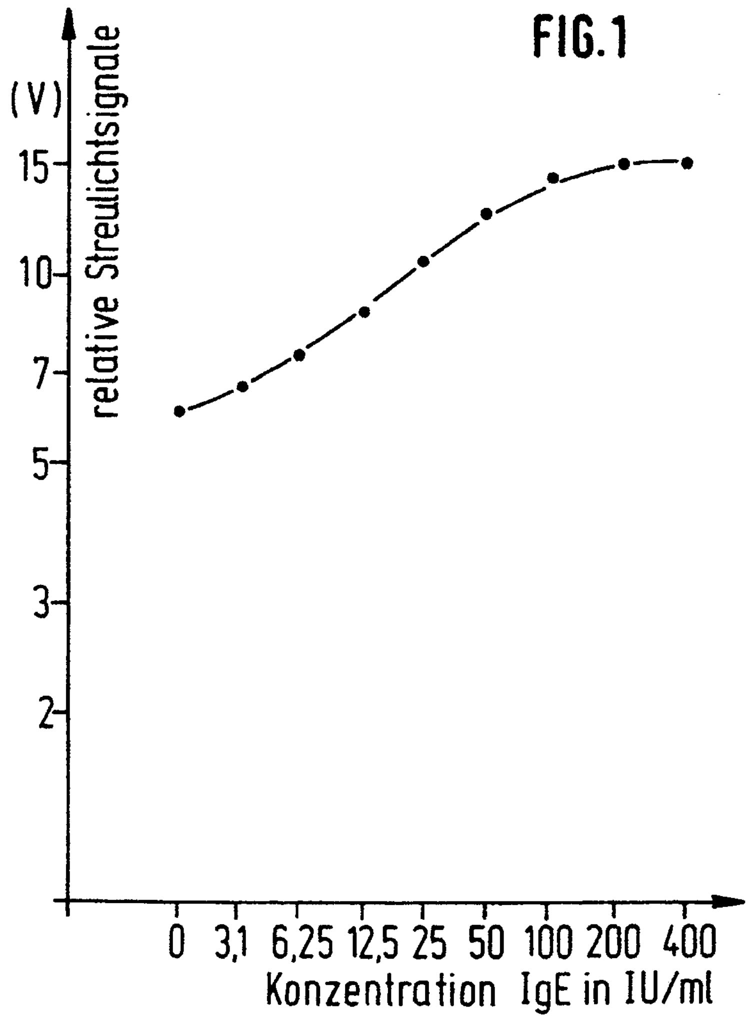
(V)
relative Streulichtsignale
15
10
7
5
3
2
0 3,1 6,25 12,5 25 50 100 200 400
Konzentration IgE in IU/ml

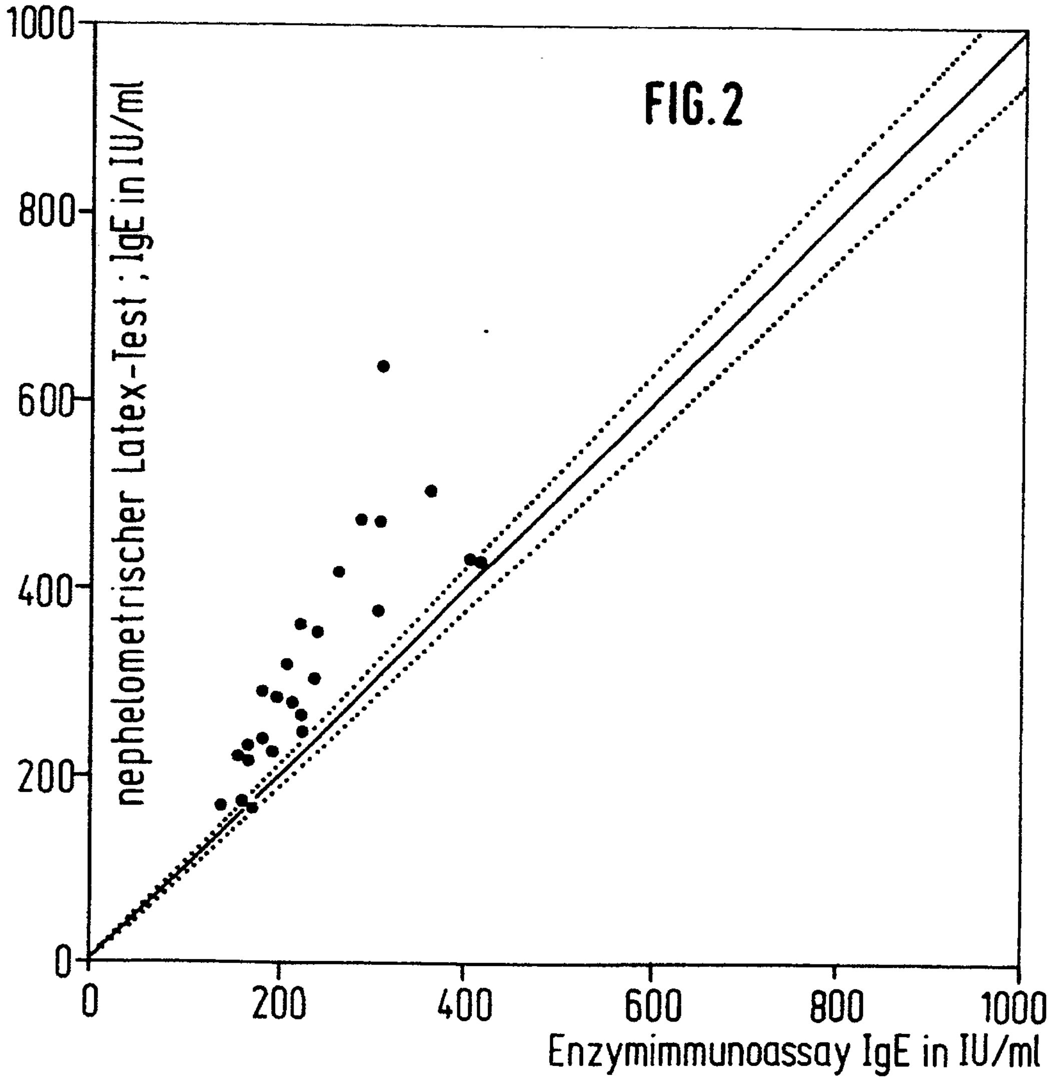
FIG. 2
1000
800
600
400
200
0
nephelometrischer Latex-Test ; IgE in IU/ml
0
200
400
600
800
1000
Enzymimmunoassay IgE in IU/ml

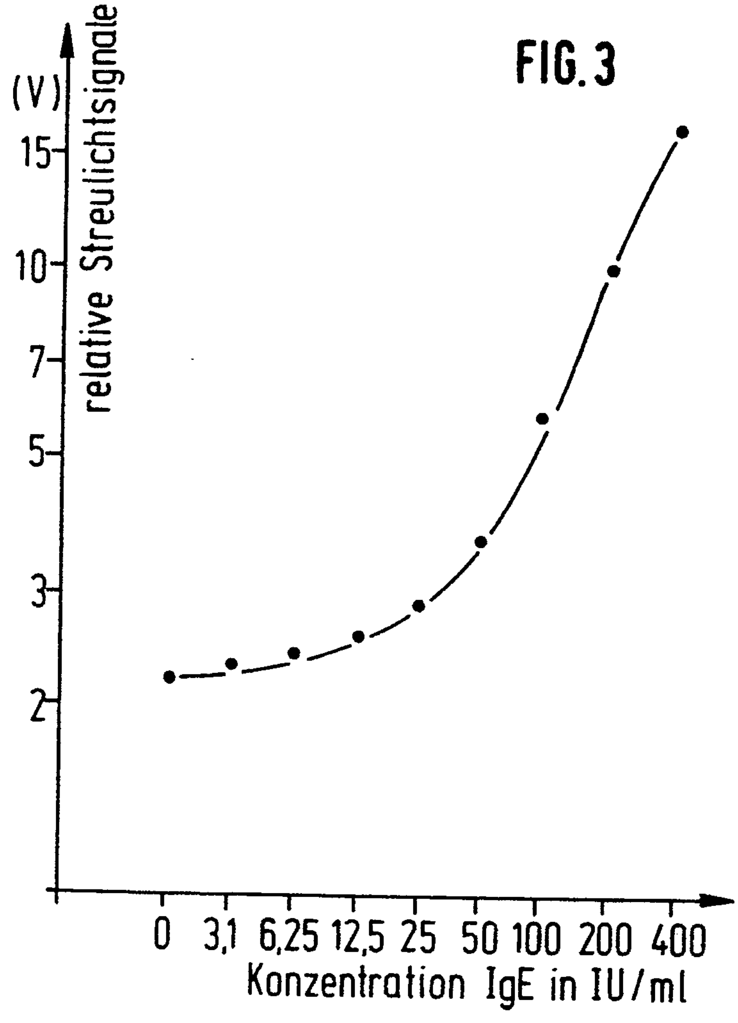

(V)
15
10
7
5
3
2
relative Streulichtsignale
0
3,1
6,25
12,5
25
50
100
200
400
Konzentration IgE in IU/ml


FIG. 3

FIG. 4

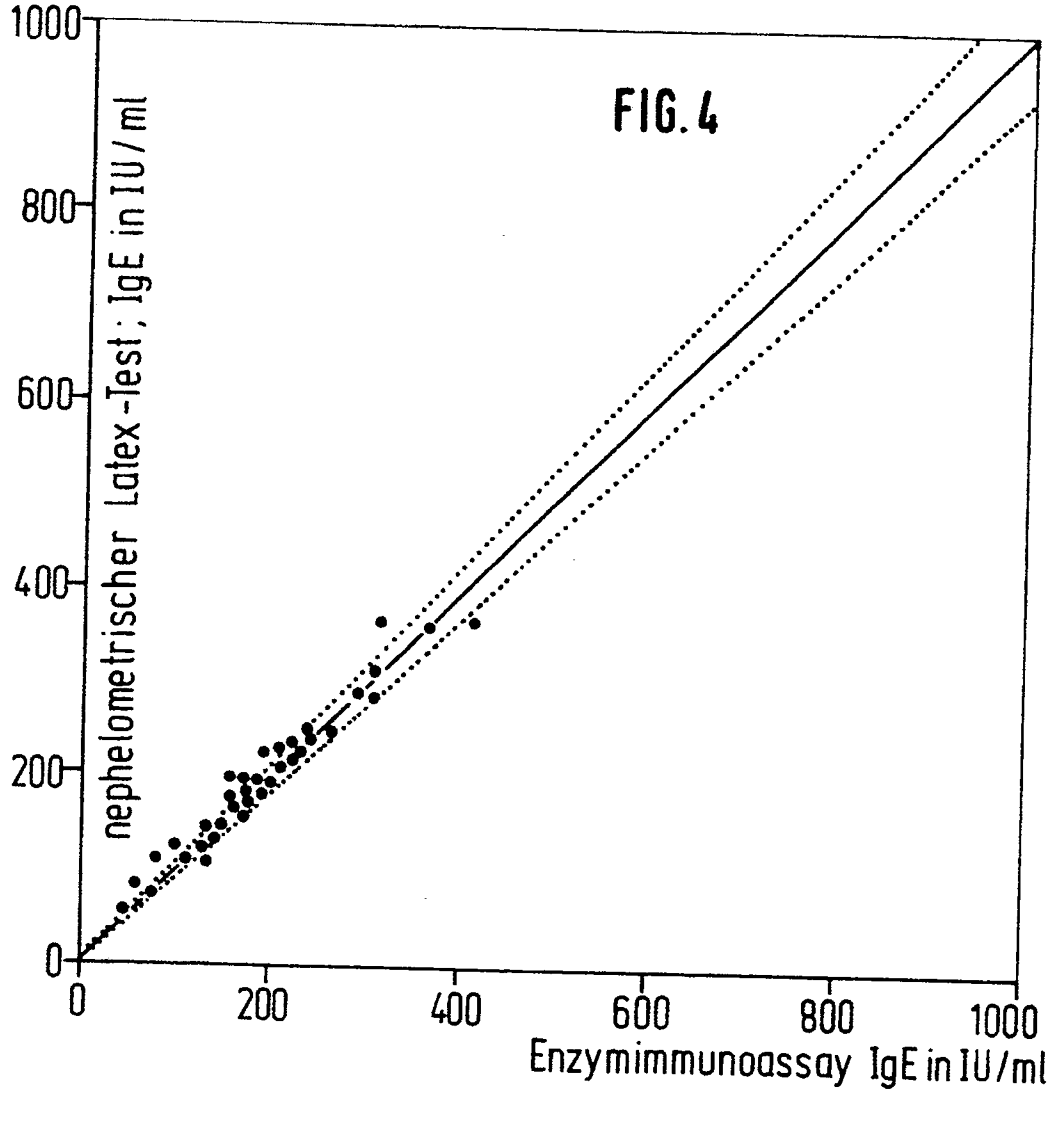
1000
800
600
400
200
0
0
200
400
600
800
1000
nephelometrischer Latex-Test; IgE in IU/ml
Enzymimmunoassay IgE in IU/ml

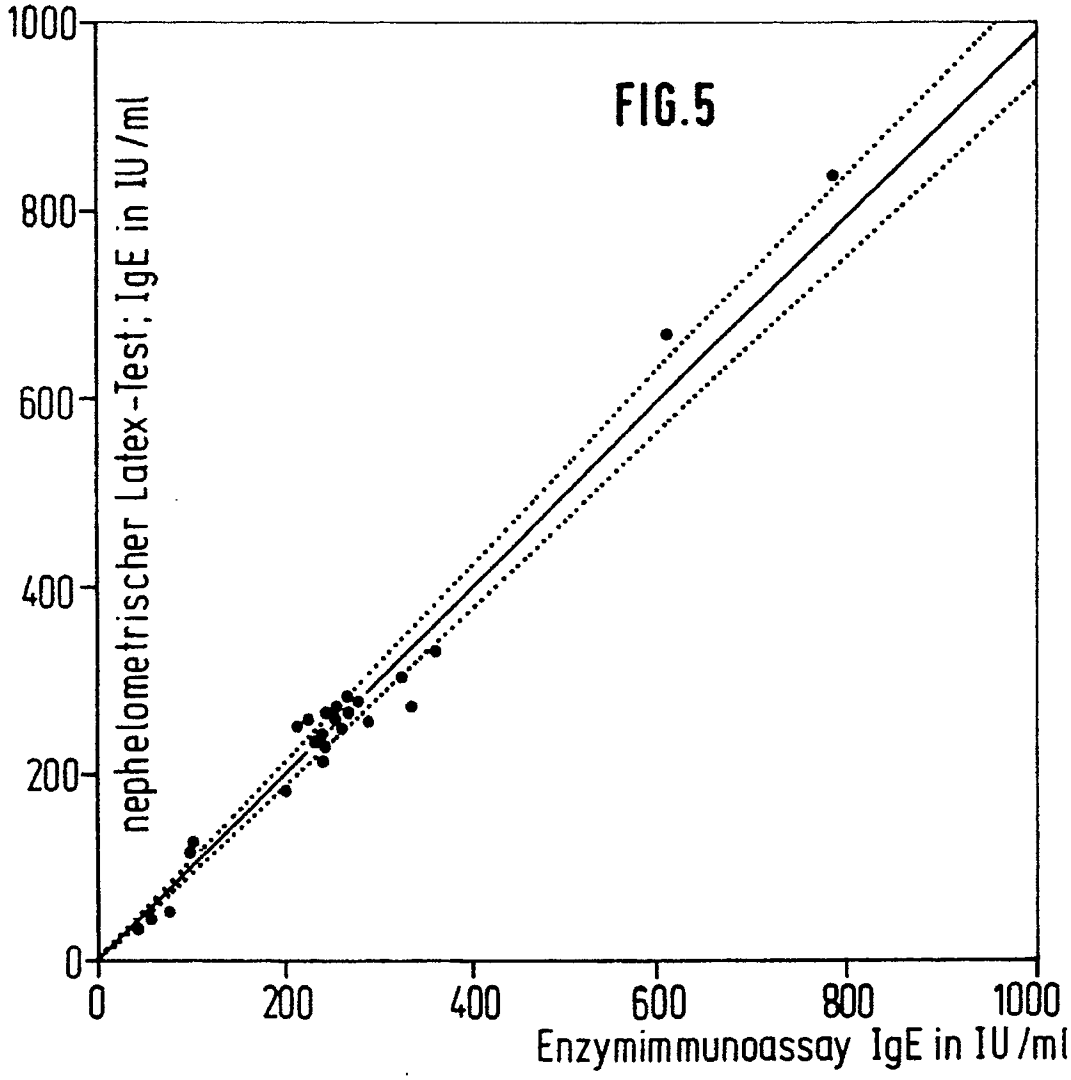
FIG.5
1000
800
600
400
200
0
nephelometrischer Latex-Test; IgE in IU/ml
0
200
400
600
800
1000
Enzymimmunoassay IgE in IU/ml